# EUROPEAN PATENT SPECIFICATION

(11) **EP 1 855 693 B1**
(45) Date of publication and mention of the grant of the patent: **29.07.2009**
(21) Application number: 05725225.6
(22) Date of filing: 07.03.2005
(51) Int. Cl.: A61K 31/70

(54) **AZITHROMYCIN POWDER FOR ORAL SUSPENSION COMPOSITIONS**
AZITHROMYCIN-PUOLVER FÜR ORALE SUSPENSIONSZUSAMMENSETZUNGEN
POUDRE D'AZITHROMYCINE POUR COMPOSITIONS ORALES SOUS FORME DE SUSPENSIONS

(43) Date of publication of application: 21.11.2007
(73) Proprietor: TEVA PHARMACEUTICAL INDUSTRIES LTD., 49131 Petah Tiqva (IL)
(72) Inventor: KOTLIAR, Eleonora Moin, 46504 Herzliya (IL); HRAKOVSKY, Julia, 48057 Rosh-Ha-Ayin (IL); TENENGAUZER, Ruth, 43393 Ra' anana (IL)
(74) Representative: Nachshen, Neil Jacob
(86) International application number: PCT/US2005/007919
(87) International publication number: WO 2006/096182

(56) References cited:
- EP-A- 0 679 400

## Description

### FIELD OF THE INVENTION

This invention relates to non-caking azithromycin powder for oral suspension (POS) compositions and methods of making such compositions.

### BACKGROUND OF THE INVENTION

Azithromycin is marked in the United States by Pfizer, Inc., under the tradename **ZITHROMAX®.**

U.S. Patent No. 5,605,889 discloses azithromycin powder for oral suspension compositions. U.S. Patent No. 4,963,531 is cited by the '889 patent and discloses in Example 2 an azithromycin powder for oral suspension. U.S. Patent No. 6,861,413 and PCT publication no. WO 2005/002592 A2, also disclose azithromycin POS compositions.

There exists a need for an improved azithromycin powder for oral suspension composition that is non-caking and a method of manufacturing such a composition.

### SUMMARY OF THE INVENTION

The present invention is directed, in one embodiment, to a non-caking azithromycin powder for oral suspension composition comprising azithromycin and a hydrated buffer. In a preferred embodiment, the hydrated buffer is a hydrate of sodium phosphate, preferably tribasic sodium phosphate dodecahydrate.

In another embodiment, the present invention is directed to a method for making a non-caking azithromycin powder for oral suspension composition comprising the steps of: a) dry mixing azithromycin and a pharmaceutically acceptable excipient to form a first mixture; b) adding to the first mixture and mixing therewith a hydrated buffer and a pharmaceutically acceptable excipient to form a second mixture; c) adding to the second mixture and mixing therewith a pharmaceutically acceptable excipient to form a third mixture; and, d) adding to the third mixture and mixing therewith a pharmaceutically acceptable excipient to form a fourth mixture. In a preferred embodiment, separate portions of a suitable diluent or sweetener, such as sucrose, are mixed in each of steps a), b), c) and d). In yet another embodiment, the present invention is directed to non-caking azithromycin powder for oral suspension compositions made by the foregoing process as more fully described hereinbelow.

### DESCRIPTION OF THE PREFERRED EMBODIMENTS

Unless otherwise indicated, the term azithromycin includes the salts, hydrates, solvates and physiologically functional derivatives and precursors thereof. The term also includes all polymorphic forms, whether crystalline or amorphous. Preferred forms of azithromycin include azithromycin dihydrate and azithromycin monohydrate hemiethanolate, such as disclosed in U.S. Patent No. 6,365,574. The azithromycin component can also be in the form of a stabilized azithromycin composition such as disclosed in U.S. Patent No. 6,764,997. The term "non-caking," used herein means that when constituted in aqueous media, for example, the formed suspension has a smooth consistency and doesn't contain any caking or clumping particles, by visual inspection. Also, the powder for oral suspension in accordance with the present invention does not cake or clump during manufacture, i.e., when mixed with excipients. Nor does it cake or clump upon storage, even under relatively humid conditions, e.g., a relative humidity of about 75% or greater and when stored for relatively long periods such as about 6 months or longer and even at elevated temperatures of about 40°C or greater, or at any combination of such humidity, time and temperature parameters. Thus, the powders for oral suspension in accordance with the present invention will remain free flowing and non-caking during typical storage and use conditions.

All percentages mentioned herein, unless otherwise indicated, are on a w/w basis, i.e., as a percentage of the total weight of the powder for oral suspension composition.

In a first embodiment, the present invention is directed to a non-caking Azithromycin powder for oral suspension composition comprising azithromycin and a hydrated buffer.

The hydrated buffer is preferably a hydrate of sodium phosphate, such as tribasic sodium phosphate dodecahydrate. Other pharmaceutically acceptable hydrated sodium phosphate buffers such as, for example, tribasic sodium phosphate hexahydrate; tribasic sodium phosphate hemihydrate; tribasic sodium phosphate octahydrate; and, dibasic sodium phosphate dihydrate and dibasic sodium phosphate heptahydrate may also be used.

The amount of the hydrated buffer present in the powder for oral suspension composition is in the range of from about 0.2% to about 2.0% w/w based on the total weight of the powder for oral suspension. More, preferably, the amount of the hydrated buffer present in the powder for oral suspension composition is in the range of from about 0.4% to about 1.2% w/w. In a particularly preferred embodiment, the hydrated buffer is tribasic sodium phosphate dodecahydrate, which is present in the powder for oral suspension composition in an amount of about 0.8% w/w.

The non-caking azithromycin powder for oral suspension compositions also may contain one or more pharmaceutically acceptable excipients. Any pharmaceutically acceptable excipient known in the art as useful in powder for oral suspension compositions may be included, such as one or more members selected from the group consisting of diluents (fillers), sweeteners, binders, suspending (thickening) agents, buffers, glidants, flavorants and colorants. An excipient can serve multiple functions, for example, as both a filler and a sweetener.

A variety of materials may be used as diluents. Examples are spray-dried or anhydrous lactose, sucrose, dextrose, mannitol, sorbitol, starch (e.g. starch 1500), cellulose (e.g. microcrystalline cellulose; AVICEL®), and others as known in the art. A preferred diluent in accordance with the present invention, which also functions as a sweetener in the powder for oral suspension compositions, is sucrose. The amount of the diluent used in the powder for oral suspension compositions of the present invention is typically in the range of from about 50% to about 98% w/w based on the total weight of the powder for oral suspension.

Sweeteners may include, for example, sucrose, glucose, aspartame, saccharin mannitol and any other pharmaceutically acceptable sweetener or combination thereof. The amount of the sweetener used in the powder for oral suspension compositions of the present invention is typically in the range of from about 50% to about 98% w/w based on the total weight of the powder for oral suspension.

Examples of binders are acacia, cellulose derivatives (such as methylcellulose and carboxymethylcellulose, hydroxypropylmethylcellulose, hydroxypropylcellulose, hydroxyethylcellulose), gelatin, glucose, dextrose, xylitol, polymethacrylates, polyvinylpyrrolidone, starch paste, sucrose, sorbitol, pregelatinized starch, gum tragacanth, alginic acids and salts thereof such as sodium alginate, magnesium aluminum silicate, polyethylene glycol, guar gum, bentonites, and the like. The amount of the binder used in the powder for oral suspension compositions of the present invention is typically in the range of from about 0.1% to about 10% w/w based on the total weight of the powder for oral suspension.

Suitable suspending agents include, for example, hydrocolloid gums such as xanthan gum, guar gum, locust bean gum, gum tragacanth, veegum, sodium alginate and the like. Alternatively, synthetic suspending agents may be used such as sodium carboxymethylcellulose, polyvinylpyrrolidone, hydroxypropylcellulose and the like. The amount of the suspending agent used in the powder for oral suspension compositions of the present invention is typically in the range of from about 0.1 % to about 10% w/w based on the total weight of the powder for oral suspension.

Dispersing agents may also be used to facilitate formation of a suspension. Among suitable dispersing agents are included, for example, colloidal silicon dioxide and other pharmaceutically acceptable dispersing agents. In preferred embodiments, a dispersing agent is not included in the powder for oral suspension. Flavorants incorporated in the powder for oral suspension compositions may be chosen from synthetic flavor oils and flavoring aromatics and/or natural oils, extracts from plants leaves, flowers, fruits, and so forth and combinations thereof. These may include cinnamon oil, oil of wintergreen, peppermint oils, clove oil, bay oil, anise oil, eucalyptus, thyme oil, cedar leaf oil, oil of nutmeg, oil of sage, oil of bitter almonds, and cassia oil. Also useful as flavors are vanilla, citrus oil, including lemon, orange, grape, lime and grapefruit, and fruit essences, including apple, banana, pear, peach, strawberry, raspberry, cherry, plum, pineapple, apricot, and so forth. Solid forms, such as spray dried forms of flavorants, are particularly useful in the powder for oral suspension compositions disclosed herein. The amount of flavoring may depend on a number of factors including the organoleptic effect desired. The amount of the flavorant used in the powder for oral suspension compositions of the present invention is typically in the range of from about 0.1 % to about 4%, based on the total weight of the powder for oral suspension.

Colorants may include titanium dioxide and/or dyes suitable for food such as those known as F. D. & C. dyes and natural coloring agents such as grape skin extract, beet red powder, beta carotene, annato, carmine, turmeric, paprika, and so forth. A colorant is an optional ingredient in the powder for oral suspension compositions disclosed herein but, when used, will generally be present in an amount up to about 0.005% to about 0.15% based on the total weight of the powder for oral suspension.

The powder for oral suspension may also contain wetting agents such as sorbitan monolaurate, polysorbate 80, and sodium lauryl sulfate; the amount of wetting agent, when employed, is typically in the range of from about 0.3% to about 3% based on the total weight of the powder for oral suspension and may also include an anti-foaming agent such as simethicone or dimethicone in amounts typically of from about 0.1% to about 4% based on the total weight of the powder for oral suspension.

The specifically mentioned suitable diluents, sweeteners, binders, suspending agents, buffers, glidants, flavorants, colorants and wetting agents in the foregoing lists are intended to be exemplary, not exhaustive, of specific excipients that may be used in the practice of the disclosed invention. It is further understood that more than one of any particular type of excipient may be used in the powder for oral suspension compositions described herein. For example, the compositions may include more than one flavorant, colorant, etc. Also, a single excipient may provide multiple functions, as mentioned hereinabove.

The powder for oral suspension compositions of the present invention are non-caking as defined hereinabove. Generally, the powder for oral suspension compositions are sold as powders, i.e., before constitution in a suspending medium. The actual suspension is typically prepared by a pharmacist. The oral suspension is thus the actual dosage form ingested by patients.

An azithromycin powder for oral suspension composition in accordance with the present invention may be supplied in bottles containing for example, 300 mg, 600 mg, 900 mg or 1200 mg azithromycin. Upon constitution in, for example, aqueous media, the suspensions will typically contain either 100 mg azithromycin/5 mL or 200 mg azithromycin/5 mL. The powders for oral suspension may also be supplied in unit dosage packets or sachets that, upon constitution in aqueous media, provide a unit dosage of azithromycin.

In another embodiment, the present invention is directed to a method for making a non-caking azithromycin powder for oral suspension composition comprising the steps of: a) dry mixing azithromycin and a pharmaceutically acceptable excipient to form a first mixture; b) adding to the first mixture and mixing therewith a hydrated buffer and a pharmaceutically acceptable excipient to form a second mixture; c) adding to the second mixture and mixing therewith a pharmaceutically acceptable excipient to form a third mixture; and, d) adding to the third mixture and mixing therewith a pharmaceutically acceptable excipient to form a fourth mixture. In a preferred embodiment, separate portions of a suitable diluent or sweetener, such as sucrose, are mixed in each of steps a), b), c) and d).

It has been surprisingly discovered that non-caking azithromycin powder for oral suspension compositions, particularly those which employ a hydrated buffer as described hereinabove, can be made in accordance with this method.

In a first step, the azithromycin is combined with pharmaceutically acceptable excipients and mixed, for example, in a high-shear mixer such as a **DIOSNA®**. This step results in a first mixture. The excipients included in the first mixture may be any of those described hereinabove. Preferred excipients include a glidant, such as colloidal silicon dioxide; a suspending agent, such as xanthan gum; a binder such as hydroxypropyl cellulose; spray-dried artificial flavorants; and a diluent/sweetening agent, such as sucrose. It is preferred that only a first portion of the total amount of sucrose or other pharmaceutically acceptable sweetener used to make the composition is mixed in the first step with any other excipients and the azithromycin. In the foregoing description, the method of making the non-caking powder for oral suspension will be described using sucrose as the primary sweetener. However, it is understood that the method may be employed with other pharmaceutically acceptable sweeteners and/or mixtures thereof. Similarly, the process is not to be construed as limited to other specific excipients named in the following discussion.

Thus, the sucrose is preferably added portion-wise to the mixture. The amount of sucrose mixed in the first step is preferably in the range of from about 1% to about 10% w/w based on the total amount of the sucrose used in the powder for oral suspension product. Preferably, the sucrose is milled using a FITZ-MILL® fitted with a 0.84 mm screen.

In a second step, the hydrated buffer is added to the high-shear mixer and mixed therein, resulting in a second mixture. Prior to adding it to the first mixture, the hydrated buffer is preferably milled along with a second part of sucrose, preferably an amount of from about 5% to about 15% w/w based on the total weight of the sucrose used in the powder for oral suspension, using, for example; a FITZ-MILL® preferably fitted with a 0.84 mm screen.

In a third step, another portion of excipients is added to the high-speed mixer and mixed therein, resulting in a third mixture. In a preferred embodiment, this portion of excipients comprises a third portion of the total amount of sucrose used to make the composition. Prior to adding it to the second mixture, the sucrose is preferably milled, for example using a FITZ-MILL®, preferably fitted with a 0.84 mm screen. The amount of sucrose mixed in this step is preferably in the range of from about 20% to about 50% w/w based on the total weight of the sucrose used in the powder for oral suspension.

In a fourth step, a remaining portion of excipients is combined with the third mixture to form a fourth mixture, which is blended in, for example a Y-Cone blender. In a preferred embodiment, this portion of excipients comprises a fourth portion of the total amount of sucrose used to make the composition. Prior to adding it to the third mixture, the sucrose is preferably sieved through a 16 mesh screen. The amount of sucrose mixed in this step is preferably in the range of from about 30% to about 70% w/w based on the total weight of the sucrose used in the powder for oral suspension.

Upon completion of blending, the final blend which comprises the powder for oral suspension has a particle size such that about 70% to about 80% of the particles are retained on a 60 mesh screen. The bulk density of the final blend will typically be about 0.75 to about 0.95 g/ml, more preferably about 0.85 g/ml.

In yet another embodiment, the present invention is directed to non-caking azithromycin powder for oral suspension compositions made by any of the foregoing processes.

The non-caking azithromycin powder for oral suspension compositions described herein may be administered to a mammal, preferably a human, in a pharmaceutically effective amount for the treatment of infection.

The present invention is further exemplified by the following non-limiting

### examples:

### EXAMPLES

### Example 1

A non-caking azithromycin powder for oral suspension composition as set forth below in Table 1 was made as described below.

**Table 1**

| **MATERIAL** | **% w/w** | **FUNCTION** |
|---|---|---|
| azithromycin (as anhydrous base) | 4.86 | active ingredient |
| colloidal silicon dioxide | 0.49 | glidant |
| sucrose | 93 | diluent, sweetening agent |
| xanthan gum | 0.16 | suspending agent |
| hydroxypropyl cellulose **KLUCEL® EF** | 0.12 | binder |
| spray dried artificial flavors | 0.49 | flavorants |
| tribasic sodium phosphate dodecahydrate | 0.8 | buffering agent |

The non-caking azithromycin powder for oral suspension formulation described in Table 1, was made as follows:
1) The azithromycin, colloidal silicon dioxide, xanthan gum, hydroxypropyl cellulose, flavor and 1-10% of the sucrose (milled with a FITZ-MILL® fitted with a 0.84 mm screen were mixed in a high-shear mixer for 2 minutes.
2) The trisodium phosphate dodecahydrate and 5-15% of the sucrose were milled with a FITZ-MILL® fitted with a 0.84 mm screen and mixed with the material from 1) in the high-shear mixer for 2 minutes.
3) A portion comprising 20-50% of the sucrose was milled through the FITZ-MILL® fitted with a 0.84 mm screen and added to the high-shear mixer and mixed for 2 minutes.
4) The remainder, comprising 40-70% of the sucrose was sieved through a 16 mesh screen, added to the mixture from 3) and blended in a Y-Cone for an additional 20 minutes.

The stability of the powder composition prepared as described above was studied by placing the powder in 60 ml HDPE bottles and storing them at room temperature/humidity, i.e., about 25°C/60% relative humidity (RH) and at accelerated conditions, i.e., about 40°C/75% RH. The bottles were capped with child resistant plastic caps. Two packaging configurations were tested for stability. One packaging configuration contained 27.5 g of powder per bottle and the other packaging configuration contained 16.5 g of powder per bottle. No caking or clumping was observed during this stability monitoring, including in powders stored for 6 months at the accelerated conditions discussed above.

The redispersibility of two batches of the powder composition prepared as described above was tested during storage at room temperature and at accelerated conditions and their suspendability was found to be successful. Redispersability testing was carried out by adding the required amount of water to the powder contained in the bottle. The bottle is shaken well and placed in an incubator at room temperature for 10 days. At the end of the 10-day period, the bottle is again shaken. The suspension was observed to have a smooth consistency with no caking or clumping observed. This demonstrates that the powder for oral suspension formulations in accordance with the present invention also do not cake or clump upon constitution in an aqueous media, as would be done prior to administration.

### Example 2

A non-caking azithromycin powder for oral suspension composition as set forth below in Table 2 was made as described below.

**Table 2**

| **MATERIAL** | **% w/w** | **FUNCTION** |
|---|---|---|
| Part I | | |
| Color FD&C Red #40 | 0.001 | colorant |
| Sucrose Caster | 3.7 | diluent, sweetening agent |
| Part II | | |
| Azithromycin monohydrate Hemiethanolate | 4.9 (as based) | active ingredient |
| Xanthan Gum | 0.16 | suspending agent |
| Hydroxypropyl cellulose | 0.12 | binder |
| Cherry Banana Vanilla (manufactured by Mane, France) | 0.5 | flavor |
| Part III | | |
| Tribasic Sodium Phosphate Dodecahydrate | 0.8 | buffering agent |
| Sucrose Caster | 7.4 | diluent, sweetening agent |
| Part IV | | |
| Sucrose Caster | 43.6 | diluent, sweetening agent |
| Part V | | |
| Sucrose Caster | 39 | diluent, sweetening agent |

| | | |
|---|---|---|
| 1) Color and Sucrose (Part I materials) were milled through the 0.84 mm screen and place into the high-shear mixer. 2) Part II materials were transferred to the high-shear mixer from step 1. 3) Part III materials were milled through the 0.84 mm screen, placed into the high-shear mixer from step 2 and mixed for 2 minutes. 4) Sucrose Caster (Part IV) was milled through the 0.84 mm screen, placed into the high-shear mixer from step 3 and mixed for 2 minutes. 5) The mixed materials from step 4 were transferred to the blender and blended with Part V Sucrose Caster sieved through the 16 mesh sieve. | | |

Redispersability testing was carried out by adding the required amount of water to the powder contained in the bottle. The bottle is shaken well and placed in an incubator at room temperature for 10 days. At the end of the 10-day period, the bottle is again shaken. The suspension was observed to have a smooth consistency with no caking or clumping observed.

## Claims

1. A non-caking azithromycin powder for oral suspension composition comprising azithromycin, a hydrated buffer and one more pharmaceutically acceptable excipients, wherein said powder is made by a process comprising the steps of:
a) dry mixing azithromycin and a pharmaceutically acceptable excipient to form a first mixture;
b) adding to the first mixture and mixing therewith a hydrated buffer and a pharmaceutically acceptable excipient to form a second mixture;
c) adding to the second mixture and mixing therewith a pharmaceutically acceptable excipient to form a third mixture; and
d) adding to the third mixture and mixing therewith a pharmaceutically acceptable excipient to form a fourth mixture.

2. The composition according to claim 1, wherein the hydrated buffer comprises a hydrate of sodium phosphate.

3. The composition according to claim 2, wherein the hydrated buffer comprises tribasic sodium phosphate dodecahydrate; tribasic sodium phosphate hexahydrate, tribasic sodium phosphate octahydrate, tribasic sodium phosphate hemihydrate, dibasic sodium phosphate dihydrate or dibasic sodium phosphate heptahydrate.

4. The composition according to claim 3, wherein the hydrated buffer comprises tribasic sodium phosphate dodecahydrate.

5. The composition according to claim 4, wherein the tribasic sodium phosphate dodecahydrate is present in an amount of from about 0.2 to about 2% w/w,

6. The composition of claim 1, wherein the pharmaceutically acceptable excipient in any of steps a), b), c) or d) comprises one or more members selected from the group consisting of diluents, sweeteners, binders, suspending agents, buffers, glidants, flavorants and colorants.

7. The azithromycin composition according to claim 6, comprising azithromycin, colloidal silicon dioxide, sucrose, xanthan gum, hydroxypropyl cellulose, artificial flavors and tribasic sodium phosphate dodecahydrate.

8. The azithromycin composition according to claim 7, comprising about 4.86% w/w azithromycin (as anhydrous base), about 0,49% w/w colloidal silicon dioxide, about 93% w/w sucrose, about 0.16% w/w xanthan gum, about 0.12% w/w hydroxypropyl cellulose, about 0.49% w/w artificial flavors and about 0.8% w/w tribasic sodium phosphate dodecahydrate.

9. The azithromycin composition according to claim 1, wherein the azithromycin is azithromycin monohydrate hemiethanolate.

10. The composition according to claim 4, wherein the tribasic sodium phosphate dodecahydrate is milled prior to adding to the first mixture,

11. The composition according to claim 6, wherein the pharmaceutically acceptable excipients in step a) comprise colloidal silicon dioxide, xanthan gum, hydroxypropyl cellulose, sucrose and flavorant.

12. The composition according to claim 11, wherein step a) comprises mixing a first portion of the total amount of sucrose used in the azithromycin composition.

13. The composition according to claim 12, wherein the amount of sucrose mixed in step a) is from about 1% to about 10% w/w based on the total weight of the sucrose.

14. The composition according to claim 11, wherein step b) comprises mixing a second portion of the total amount of sucrose used in the azithromycin composition.

15. The composition according to claim 14, wherein the amount of sucrose mixed in step b) is from about 5% to about 15% w/w based on the total weight of the sucrose.

16. The composition according to claim 11, wherein step c) comprises mixing a third portion of the total amount of sucrose used in the azithromycin composition.

17. The composition according to claim 16, wherein the amount of sucrose mixed in step c) is from about 20% to about 50% w/w based on the total weight of the sucrose.

18. The composition according to claim 11, wherein step d) comprises mixing a fourth and remaining portion of the total amount of sucrose used in the azithromycin composition.

19. The composition according to claim 18, wherein the amount of sucrose mixed in step d) is from about 30% to about 70% w/w based on the total weight of the sucrose.

20. The composition according to claim 1, wherein the azithromycin comprises azithromycin monohydrate hemiethanolate.

21. A non-caking azithromycin powder for oral suspension composition comprising azithromycin, a hydrated buffer and a pharmaceutically acceptable excipient, wherein said powder is made by a process comprising the steps of mixing azithromycin and the pharmaceutically acceptable excipient to form a first mixture and mixing the first mixture with a hydrated buffer to form a second mixture.

22. The composition according to claim 21, wherein the process further comprises adding to the second mixture and mixing therewith a pharmaceutically acceptable excipient to form a third mixture.

23. The composition according to claim 22, wherein the process further comprises adding to the third mixture and mixing therewith a pharmaceutically acceptable excipient to form a fourth mixture.

24. A non-caking azithromycin powder for oral suspension composition comprising azithromycin monohydrate hemiethanolate and a hydrated buffer.

25. The composition according to claim 24, wherein the hydrated buffer comprises a hydrate of sodium phosphate.

26. The composition according to claim 25, wherein the hydrated buffer comprises tribasic sodium phosphate dodecahydrate, tribasic sodium phosphate hexahydrate, tribasic sodium phosphate octahydrate, tribasic sodium phosphate hemihydrate, dibasic sodium phosphate dihydrate or dibasic sodium phosphate heptahydrate.

27. The composition according to claim 26, wherein the hydrated buffer comprises tribasic sodium phosphate dodecahydrate.

28. The composition according to claim 27, wherein the tribasic sodium phosphate dodecahydrate is present in an amount of from about 0.2 to about 2% w/w.

29. The composition according to any of claims 24 to 28, further comprising a pharmaceutically acceptable excipient,

30. The composition of claim 29, wherein the pharmaceutically acceptable excipient comprises one or more members selected from the group consisting of diluents, sweeteners, binders, suspending agents, buffers, glidants, flavorants and colorants.

31. The azithromycin composition according to claim 30, comprising azithromycin monohydrate hemiethanolate, colloidal silicon dioxide, sucrose, xanthan gum, hydroxypropyl cellulose, artificial flavors and tribasic sodium phosphate dodecahydrate.

32. A method for making a non-caking azithromycin powder for oral suspension, wherein said method is as defined in any of claims 1 to 23.

## Patentansprüche

1. Zusammensetzung eines nicht zusammenbackenden Azithromycin-Pulvers für eine orale Suspension, welche Azithromycin, einen hydrierten Puffer und einen oder mehrere pharmazeutisch verträgliche Hilfsstoffe umfaßt, wobei das Pulver durch ein Verfahren hergestellt wird, welches die Stufen umfaßt:
a) Trockenmischen von Azithromycin und einem pharmazeutisch verträglichen Hilfsstoff unter Bildung eines ersten Gemischs,
b) Zugeben eines hydrierten Puffers und eines pharmazeutisch verträglichen Hilfsstoffs zu dem ersten Gemisch und Vermischen mit diesem unter Bildung eines zweiten Gemischs,
c) Zugeben eines pharmazeutisch verträglichen Hilfsstoffs zu dem zweiten Gemisch und Vermischen mit diesem unter Bildung eines dritten Gemischs und
d) Zugeben eines pharmazeutisch verträglichen Hilfsstoffs zu dem dritten Gemisch und Vermischen mit diesem unter Bildung eines vierten Gemischs.

2. Zusammensetzung nach Anspruch 1, wobei der hydrierte Puffer ein Hydrat von Natriumphosphat umfaßt.

3. Zusammensetzung nach Anspruch 2, wobei der hydrierte Puffer dreibasiges Natriumphosphatdodecahydrat, dreibasiges Natriumphosphathexahydrat, dreibasiges Natriumphosphatoctahydrat, dreibasiges Natriumphosphathemihydrat, zweibasiges Natriumphosphatdihydrat oder zweibasiges Natriumphosphatheptahydrat umfaßt.

4. Zusammensetzung nach Anspruch 3, wobei der hydrierte Puffer dreibasiges Natriumphosphatdodecahydrat umfaßt.

5. Zusammensetzung nach Anspruch 4, wobei das dreibasige Natriumphosphatdodecahydrat in einer Menge von etwa 0,2 bis etwa 2% m/m vorliegt.

6. Zusammensetzung nach Anspruch 1, wobei der pharmazeutisch verträgliche Hilfsstoff in einer beliebigen der Stufen a), b), c) oder d) einen oder mehrere Bestandteile umfaßt, die aus der Gruppe ausgewählt sind, bestehend aus Verdünnungsmitteln, Süßungsmitteln, Bindemitteln, Suspendiermitteln, Puffern, Gleitmitteln, Aromastoffen und Farbstoffen.

7. Azithromycin-Zusammensetzung nach Anspruch 6, welche Azithromycin, kolloidales Siliciumdioxid, Saccharose, Xanthangummi, Hydroxypropylzellulose, künstliche Aromen und dreibasiges Natriumphosphatdodecahydrat umfaßt.

8. Azithromycin-Zusammensetzung nach Anspruch 7, welche etwa 4,86 Gew.-% m/m Azithromycin (als wasserfreie Base), etwa 0,49% m/m kolloidales Siliciumdioxid, etwa 93% m/m Saccharose, etwa 0,16% m/m Xanthangummi, etwa 0,12% m/m Hydroxypropylzellulose, etwa 0,49% m/m künstliche Aromen und etwa 0,8% m/m dreibasiges Natriumphosphatdodecahydrat umfaßt.

9. Azithromycin-Zusammensetzung nach Anspruch 1, wobei das Azithromycin Azithromycinmonohydrat-hemiethanolat ist.

10. Zusammensetzung nach Anspruch 4, wobei das dreibasige Natriumphosphatdodecahydrat vor der Zugabe zu dem ersten Gemisch gemahlen wird.

11. Zusammensetzung nach Anspruch 6, wobei die pharmazeutisch verträglichen Hilfsstoffe in Stufe a) kolloidales Siliciumdioxid, Xanthangummi, Hydroxypropylzellulose, Saccharose und Aromastoff umfassen.

12. Zusammensetzung nach Anspruch 11, wobei Stufe a) das Mischen eines ersten Teils der Gesamtmenge an Saccharose, die in der Azithromycin-Zusammensetzung verwendet wird, umfaßt.

13. Zusammensetzung nach Anspruch 12, wobei die Menge an Saccharose, die in Stufe a) gemischt wird, etwa 1% bis etwa 10% m/m, basierend auf dem Gesamtgewicht der Saccharose, beträgt.

14. Zusammensetzung nach Anspruch 11, wobei Stufe b) das Mischen eines zweiten Teils der Gesamtmenge an Saccharose, die in der Azithromycin-Zusammensetzung verwendet wird, umfaßt.

15. Zusammensetzung nach Anspruch 14, wobei die Menge an Saccharose, die in Stufe b) gemischt wird, etwa 5% bis etwa 15% m/m, basierend auf dem Gesamtgewicht der Saccharose, beträgt.

16. Zusammensetzung nach Anspruch 11, wobei Stufe c) das Mischen eines dritten Teils der Gesamtmenge an Saccharose, die in der Azithromycin-Zusammensetzung verwendet wird, umfaßt.

17. Zusammensetzung nach Anspruch 16, wobei die Menge an Saccharose, die in Stufe c) gemischt wird, etwa 20% bis etwa 50% m/m, basierend auf dem Gesamtgewicht der Saccharose, beträgt.

18. Zusammensetzung nach Anspruch 11, wobei Stufe d) das Mischen eines vierten und verbleibenden Teils der Gesamtmenge an Saccharose, die in der Azithromycin-Zusammensetzung verwendet wird, umfaßt.

19. Zusammensetzung nach Anspruch 18, wobei die Menge an Saccharose, die in Stufe d) gemischt wird, etwa 30% bis etwa 70% m/m, basierend auf dem Gesamtgewicht der Saccharose, beträgt.

20. Zusammensetzung nach Anspruch 1, wobei das Azithromycin Azithromycinmonohydrat-hemiethanolat umfaßt.

21. Zusammensetzung eines nicht zusammenbackenden Azithromycin-Pulvers für eine orale Suspension, welche Azithromycin, einen hydrierten Puffer und einen pharmazeutisch verträglichen Hilfsstoff umfaßt, wobei das Pulver durch ein Verfahren hergestellt wird, welches die Stufen des Mischens von Azithromycin und des pharmazeutisch verträglichen Hilfsstoffs unter Bildung eines ersten Gemischs und das Mischen des ersten Gemischs mit einem hydrierten Puffer unter Bildung eines zweiten Gemischs umfaßt.

22. Zusammensetzung nach Anspruch 21, wobei das Verfahren weiterhin das Zugeben eines pharmazeutisch verträglichen Hilfsstoffs zu dem zweiten Gemisch und das Vermischen mit diesem unter Bildung eines dritten Gemischs umfaßt.

23. Zusammensetzung nach Anspruch 22, wobei das Verfahren weiterhin das Zugeben eines pharmazeutisch verträglichen Hilfsstoffs zu dem dritten Gemisch und das Vermischen mit diesem unter Bildung eines vierten Gemischs umfaßt.

24. Zusammensetzung eines nicht zusammenbackenden Azithromycin-Pulvers für eine orale Suspension, welche Azithromycinmonohydrat-hemiethanolat und einen hydrierten Puffer umfaßt.

25. Zusammensetzung nach Anspruch 24, wobei der hydrierte Puffer ein Hydrat von Natriumphosphat umfaßt.

26. Zusammensetzung nach Anspruch 25, wobei der hydrierte Puffer dreibasiges Natriumphosphatdodecahydrat, dreibasiges Natriumphosphathexahydrat, dreibasiges Natriumphosphatoctahydrat, dreibasiges Natriumphosphathemihydrat, zweibasiges Natriumphosphatdihydrat oder zweibasiges Natriumphosphatheptahydrat umfaßt.

27. Zusammensetzung nach Anspruch 26, wobei der hydrierte Puffer dreibasiges Natriumphosphatdodecahydrat umfaßt.

28. Zusammensetzung nach Anspruch 27, wobei das dreibasige Natriumphosphatdodecahydrat in einer Menge von etwa 0,2 bis etwa 2% m/m vorliegt.

29. Zusammensetzung nach einem der Ansprüche 24 bis 28, welche weiterhin einen pharmazeutisch verträglichen Hilfsstoff umfaßt.

30. Zusammensetzung nach Anspruch 29, wobei der pharmazeutisch verträgliche Hilfsstoff einen oder mehrere Bestandteile umfaßt, die aus der Gruppe ausgewählt sind, bestehend aus Verdünnungsmitteln, Süßungsmitteln, Suspendiermitteln, Puffern, Gleitmitteln, Aromastoffen und Farbstoffen.

31. Azithromycin-Zusammensetzung nach Anspruch 30, welche Azithromycinmonohydrat-hemiethanolat, kolloidales Siliciumdioxid, Saccharose, Xanthangummi, Hydroxypropylzellulose, künstliche Aromen und dreibasiges Natriumphosphatdodecahydrat umfaßt.

32. Verfahren zum Herstellen eines nicht zusammenbackenden Azithromycin-Pulvers für eine orale Suspension, wobei das Verfahren wie in einem der Ansprüche 1 bis 23 definiert ist.

## Revendications

1. Poudre d'azithromycine non agglutinante pour composition orale sous forme de suspension comprenant de l'azithromycine, un tampon hydraté et un ou plusieurs excipients pharmaceutiquement acceptables, dans laquelle ladite poudre est fabriquée selon un procédé comprenant les étapes consistant à :
a) mélanger à sec de l'azithromycine et un excipient pharmaceutiquement acceptable pour former un premier mélange ;
b) ajouter un tampon hydraté et un excipient pharmaceutiquement acceptable au premier mélange et les mélanger pour former un deuxième mélange ;
c) ajouter un excipient pharmaceutiquement acceptable au deuxième mélange et les mélanger pour former un troisième mélange ; et
d) ajouter un excipient pharmaceutiquement acceptable au troisième mélange et les mélanger pour former un quatrième mélange.

2. Composition selon la revendication 1, dans lequel le tampon hydraté comprend un hydrate de phosphate de sodium.

3. Composition selon la revendication 2, dans laquelle le tampon hydraté comprend le dodécahydrate de phosphate de sodium tribasique, l'hexahydrate de phosphate de sodium tribasique, l'octahydrate de phosphate de sodium tribasique, l'hémihydrate de phosphate de sodium tribasique, le dihydrate de phosphate de sodium dibasique ou l'heptahydrate de phosphate de sodium dibasique.

4. Composition selon la revendication 3, dans laquelle le tampon hydraté comprend le dodécahydrate de phosphate de sodium tribasique.

5. Composition selon la revendication 4, dans laquelle le dodécahydrate de phosphate de sodium tribasique est présent en une quantité allant d'environ 0,2 à environ 2 % p/p.

6. Composition selon la revendication 1, dans laquelle l'excipient pharmaceutiquement acceptable de l'une quelconque des étapes a), b), c) ou d) comprend un ou plusieurs éléments choisis dans le groupe consistant en les diluants, les édulcorants, les liants, les agents de suspension, les tampons, les glissants, les essences et les colorants.

7. Composition d'azithromycine selon la revendication 6, comprenant de l'azithromycine, de la silice colloïdale, du saccharose, de la gomme de xanthane, de l'hydroxypropylcellulose, des arômes artificiels et du dodécahydrate de phosphate de sodium tribasique.

8. Composition d'azithromycine selon la revendication 7, comprenant environ 4,86 % p/p d'azithromycine (comme base anhydre), environ 0,49 % p/p de silice colloïdale, environ 93 % p/p de saccharose, environ 0,16 % p/p de gomme de xanthane, environ 0,12 % p/p d'hydroxypropylcellulose, environ 0,49 % p/p d'arômes artificiels et environ 0,8 % p/p de dodécahydrate de phosphate de sodium tribasique.

9. Composition d'azithromycine selon la revendication 1, dans laquelle l'azithromycine est le monohydrate hémiéthanolate d'azithromycine.

10. Composition selon la revendication 4, dans laquelle le dodécahydrate de phosphate de sodium tribasique est broyé avant l'ajout au premier mélange.

11. Composition selon la revendication 6, dans laquelle les excipients pharmaceutiquement acceptables de l'étape a) comprennent de la silice colloïdale, de la gomme de xanthane, de l'hydroxypropylcellulose, du saccharose et une essence.

12. Composition selon la revendication 11, dans laquelle l'étape a) comprend le mélange d'une première partie de la quantité totale de saccharose utilisée dans la composition d'azithromycine.

13. Composition selon la revendication 12, dans laquelle la quantité de saccharose mélangée lors de l'étape a) va d'environ 1 % à environ 10 % p/p par rapport au poids total du saccharose.

14. Composition selon la revendication 11, dans laquelle l'étape b) comprend le mélange d'une deuxième partie de la quantité totale de saccharose utilisée dans la composition d'azithromycine.

15. Composition selon la revendication 14, dans laquelle la quantité de saccharose mélangée lors de l'étape b) va d'environ 5 % à environ 15 % p/p par rapport au poids total du saccharose.

16. Composition selon la revendication 11, dans laquelle l'étape c) comprend le mélange d'une troisième partie de la quantité totale de saccharose utilisée dans la composition d'azithromycine.

17. Composition selon la revendication 16, dans laquelle la quantité de saccharose mélangée lors de l'étape c) va d'environ 20 % à environ 50 % p/p par rapport au poids total du saccharose.

18. Composition selon la revendication 11, dans laquelle l'étape d) comprend le mélange d'une quatrième partie restante de la quantité totale de saccharose utilisée dans la composition d'azithromycine.

19. Composition selon la revendication 18, dans laquelle la quantité de saccharose mélangée lors de l'étape d) va d'environ 30 % à environ 70 % p/p par rapport au poids total du saccharose.

20. Composition selon la revendication 1, dans laquelle l'azithromycine comprend le monohydrate hémiéthanolate d'azithromycine.

21. Poudre d'azithromycine non agglutinante pour composition orale sous forme de suspension comprenant de l'azithromycine, un tampon hydraté et un excipient pharmaceutiquement acceptable, dans laquelle ladite poudre est fabriquée selon un procédé comprenant les étapes de mélange de l'azithromycine et de l'excipient pharmaceutiquement acceptable pour former un premier mélange et de mélange du premier mélange avec un tampon hydraté pour former un deuxième mélange.

22. Composition selon la revendication 21, dans laquelle le procédé comprend en outre l'ajout d'un excipient pharmaceutiquement acceptable au deuxième mélange et leur mélange pour former un troisième mélange.

23. Composition selon la revendication 22, dans laquelle le procédé comprend en outre l'ajout d'un excipient pharmaceutiquement acceptable au troisième mélange et leur mélange pour former un quatrième mélange.

24. Poudre d'azithromycine non agglutinante pour composition orale sous forme de suspension comprenant du monohydrate hémiéthanolate d'azithromycine et un tampon hydraté.

25. Composition selon la revendication 24, dans laquelle le tampon hydraté comprend un hydrate de phosphate de sodium.

26. Composition selon la revendication 25, dans laquelle le tampon hydraté comprend le dodécahydrate de phosphate de sodium tribasique, l'hexahydrate de phosphate de sodium tribasique, l'octahydrate de phosphate de sodium tribasique, l'hémihydrate de phosphate de sodium tribasique, le dihydrate de phosphate de sodium dibasique ou l'heptahydrate de phosphate de sodium dibasique.

27. Composition selon la revendication 26, dans laquelle le tampon hydraté comprend le dodécahydrate de phosphate de sodium tribasique.

28. Composition selon la revendication 27, dans laquelle le dodécahydrate de phosphate de sodium tribasique est présent en une quantité allant d'environ 0,2 à environ 2 % p/p.

29. Composition selon l'une quelconque des revendications 24 à 28, comprenant en outre un excipient pharmaceutiquement acceptable.

30. Composition selon la revendication 29, dans laquelle l'excipient pharmaceutiquement acceptable comprend un ou plusieurs éléments choisis dans le groupe consistant en les diluants, les édulcorants, les liants, les agents de suspension, les tampons, les glissants, les essences et les colorants.

31. Composition d'azithromycine selon la revendication 30, comprenant du monohydrate hémiéthanolate d'azithromycine, de la silice colloïdale, du saccharose, de la gomme de xanthane, de l'hydroxypropylcellulose, des arômes artificiels et du dodécahydrate de phosphate de sodium tribasique.

32. Procédé de préparation d'une poudre d'azithromycine non agglutinante pour suspension orale, dans lequel ledit procédé est tel que défini selon l'une quelconque des revendications 1 à 23.
